# EUROPEAN PATENT SPECIFICATION

(11) **EP 2 945 622 B1**
(45) Date of publication and mention of the grant of the patent: **30.11.2022**
(21) Application number: 14703038.1
(22) Date of filing: 20.01.2014
(51) Int. Cl.: A61K 31/198, A61K 31/37, A61P 21/06, A61P 5/26, A61P 19/10, G01N 33/48, A61K 31/00, A61K 45/06, A61K 31/568, A61K 31/19

(54) **COMPOSITION COMPRISING UROLITHIN B AND TESTOSTERONE, LEUCINE AND/OR CREATINE FOR MUSCLE GROWTH**
ZUSAMMENSETZUNG MIT UROLITHIN B UND TESTOSTERON, LEUCIN UND/ODER KREATIN FÜR MUSKELWACHSTUM
COMPOSITION COMPRENANT DE L'UROLITHINE B ET DE LA TESTOSTÉRONE, DE LA LEUCINE ET/OU DE LA CRÉATINE POUR LA CROISSANCE MUSCULAIRE

(30) Priority: 18.01.2013 EP 13151864
(43) Date of publication of application: 25.11.2015
(73) Proprietor: Mans, Jean-Pierre, 1410 Waterloo (BE)
(72) Inventor: PRIEM, Fabian, B-1310 La Hulpe (BE); RODRIGUEZ, Julie, F-13110 Port de Bouc (FR); FRANCAUX, Marc, B-1348 Louvain-la-Neuve (BE); FAUCHET, Fabienne, B-1435 Corbais (BE)
(74) Representative: Peel, James Peter
(86) International application number: PCT/EP2014/051047
(87) International publication number: WO 2014/111580

(56) References cited:
- WO-A1-2008/016554
- WO-A2-2012/088519
- WO-A2-2014/004902
- US-A1- 2013 017 283
- SHANON L CASPERSON ET AL: "Leucine supplementation chronically improves muscle protein synthesis in older adults consuming the RDA for protein", CLINICAL NUTRITION, CHURCHILL LIVINGSTONE, LONDON, GB, vol. 31, no. 4, 26 January 2012 (2012-01-26), pages 512-519, XP028448746, ISSN: 0261-5614, DOI: 10.1016/J.CLNU.2012.01.005 [retrieved on 2012-01-31]
- Prakash S Bisen ET AL: "Ellagic Acid - Chemopreventive Role in Oral Cancer", Journal of Cancer Science & Therapy, vol. 04, no. 02, 1 January 2012 (2012-01-01), XP055159476, DOI: 10.4172/1948-5956.1000106
- Juan Carlos Esp?n ET AL: "Iberian Pig as a Model To Clarify Obscure Points in the Bioavailability and Metabolism of Ellagitannins in Humans", Journal of Agricultural and Food Chemistry, vol. 55, no. 25, 1 December 2007 (2007-12-01), pages 10476-10485, XP055159485, ISSN: 0021-8561, DOI: 10.1021/jf0723864

## Description

### Field of the invention

The present invention relates to a composition comprising urolithin B and a compound selected from the group consisting of testosterone, leucine and/or creatine for enhancing muscle growth and/or as androgen receptor agonist.

### Background of the invention and state of the art

Ageing is often associated with reduced muscle size and strength. Several possible mechanisms have been proposed, including mitochondrial somatic mutations, oxidative stress and low-grade inflammation. However, recent studies have touted the beneficial effect of high dose antioxidant supplementation on muscle capacities as it seems that muscle under chronic oxidative stress, but not under effort-induced oxidative stress, benefit from antioxidant supplements.

Polyphenols are well known for their antioxidant and/or anti-inflammatory properties.

One of the sources of polyphenols is pomegranate extract. During digestion, various constituents of the pomegranate extract are converted by the gut flora into urolithin C, which is then transformed into urolithin D, A, and, finally, into urolithin B. Consequently, small amounts of urolithin B can be found in the blood of human patients fed with pomegranate extract; urolithin B amount in the blood is much lower than urolithin A amount.

Beside being of lower abundance in the blood, at least in comparison with urolithin A (Gonzalez-Barrio et al., 2012), the antioxidant and anti-inflammatory properties of urolithin B are much weaker than the other related compounds, such as urolithins A, C and D, as well as a whole polyphenol extract.

Beside the antioxidant action, ellagitannin-derived compounds such as urolithin A or B, have been shown to have direct anti-cancer activity, at least on breast and on prostate cancer cell lines, probably due to inhibition of testosterone-induced cell proliferation of MCF-7 cells and/or on inhibition of aromatase activity (Adams et al., 2010).

WO2012/088519 discloses and demonstrates in the examples a composition comprising Urolithin A which may be used for a variety of therapeutic applications including improving or maintain muscle performance or for use in the treatment of muscle or neuromuscular disease such as myopathy, but it does not demonstrate the use of Urolithin B. The usefulness of Urolithin B for the above diseases is also evoked.

S. Casperson et al. disclose in Clin. Nutrit., 2012, 512-519, that leucine is effective in increasing muscle mass.

### Summary of the invention

The present invention is defined by the claims. Any subject matter falling outside the scope of the claims is provided for information purposes only.

The present invention covers a composition (preferably a food composition (thus also including a beverage), a feed composition and/or a pharmaceutical composition) comprising a sufficient amount of urolithin B, as defined in the claims.

This composition further comprises a sufficient amount of a compound (synergizing with urolithin B) selected from the group consisting of leucine, creatine, and/or of testosterone. By "synergising", it is preferably meant that, upon addition of a sufficient amount of urolithin B, a lower amount of the other (synergic) compound is given than normally, while allowing the same effect (e.g. on muscle size), or that, upon addition of the other (synergic) compound at the regular dose, the presence of sufficient amount of urolithin B allows an increased effect (e.g. on muscle size).

A suitable composition comprises at least 0.1% (preferably at least 0.5% or even at least 10%) of urolithin B (% by weight urolithin B : dry weight composition), and a sufficient amount of leucine (preferably at least 5% weight, more preferably at least 10% weight, but less than 100% weight: dry weight composition).

A related (alternative) suitable disclosed composition comprises at least 10% (preferably at least 15 % or even at least 50%) of urolithin B (weight urolithin B: dry weight composition), and a sufficient amount of leucine (preferably at least 5%, more preferably at least 10%, but less than 100% (weight: dry weight composition)).

Another related (alternative) suitable composition comprises at least 0.1% of urolithin B (% by weight urolithin B:dry weight composition), and a sufficient amount of leucine.

Alternatively, these compositions comprising urolithin B comprise a sufficient amount of creatine.

Preferably, these compositions comprising urolithin B and leucine further comprise at least 100 mg of creatine.

Preferably, these compositions (comprising urolithin B, leucine (and possibly creatine and/or other synergic compound) are edible (and/or is a beverage).

Preferably, these compositions further comprise at least an additive selected from the group consisting of vitamin D, magnesium, lactoferrin, ursolic acid and mixture thereof.

Preferably, these edible compositions (and/or beverage) have an amount of urolithin B that is sufficient for obtaining urolithin B content (i.e. not urolithin A) of at least - 100 nM) in the blood of a human being or of an animal (preferably a mammal, more preferably selected from the group consisting of pigs, sheep, cattle, dogs and horse), wherein preferably this mammal (human being or animal) has taken the edible composition several times (e.g. 2-4) per day or per week (e.g. 2-14) for at least 3 or even at least 6 months.

A related aspect of the present invention is a pharmaceutical composition comprising urolithin B , wherein the amount of the urolithin B is of at least 1 mg and/or of at least 0.1% (preferably at least 10%; weight urolithin B : dry weight pharmaceutical composition) and an adequate or suitable pharmaceutical carrier or diluent, as defined in the claims.

This pharmaceutical composition can be edible or is non-edible (an injectable composition, a composition for topical administration and/or incorporated into a (dermal) patch).

This (edible) pharmaceutical composition further comprises at least 0.25 g (preferably at least 0.5 g) of leucine and/or at least 0.1 g of creatine.

Optionally, this (non-edible) pharmaceutical composition further comprises at least 0.01 % of a compound selected from the group consisting of capsaicin, menthol, trolamine salicylate, camphor, methylsalicylate (weight compound: dry weight pharmaceutical composition). This (edible) pharmaceutical composition may also comprise (a sufficient amount of) hydroxymethyl butyrate. By sufficient amount of hydroxymethyl butyrate, it is preferably meant between 0.5 g and 5 g per day to a human adult weighting from about 60 kg to about 100 kg (e.g. a human patient affected by a myopathy or an elder human patient), more preferably between 1 g and 3 g, still more preferably between 1.5 g and 2 g. The skilled person can easily adapt (downwards, or even upwards) such dose to non-human mammal or to humans of lower weight (such as infants or severely diseased patients).

Another related aspect of the present invention is urolithin B for a therapeutic use as androgen receptor agonist for the uses as defined in the claims.

Another related aspect of the present invention is urolithin B in the claimed compositions for use in enhancing muscle growth and/or size in (an elder) human patient or in the prevention or treatment of myopathy.

Another related non-claimed aspect of the present disclosure is urolithin B for use in the prevention or treatment of osteoporosis in a human patient (preferably having been diagnosed as suffering or as risk of suffering of osteoporosis).

Another related aspect of the present disclosure is urolithin B for (a therapeutic) use in reducing muscle weight loss in a patient (e.g. an elder patient and/or a patient suffering of myopathy and/or a patient having been immobilized and/or a patient at risk of losing muscle mass).

Another related aspect of the present disclosure is urolithin B and a compound in synergy for muscle growth and/or repair for (a therapeutic) use in reducing muscle weight loss in a patient (e.g. an elder patient and/or a patient suffering of myopathy and/or a patient having been immobilized and/or a patient at risk of losing muscle mass).

Another related aspect of the present invention is a kit of parts comprising urolithin B, and another compound testosterone and hydroxymethyl butyrate in synergy for muscle growth and/or repair.

Another related aspect of the present invention is the non-therapeutic use of urolithin B as androgen receptor agonist together with testosterone and hydroxymethyl butyrate for enhancing muscle size in a mammal.

Another related aspect of the present disclosure is the non-therapeutical use of urolithin B for increasing muscle mass of a mammal (such as a human being or an animal selected from the group consisting of pigs, sheep, cattle, dogs, and horse or camel), or poultry or fish).

This use of urolithin B is in synergy with another compound which is testosterone and hydroxymethyl butyrate for increasing muscle mass of a mammal.

Another related non-claimed aspect of the present disclosure is a process to identify a non-steroid (natural) androgen receptor agonist, comprising the steps of: growing myoblast (mammalian; non-human embryonic) cells, applying compositions putatively comprising an androgen agonist to these (myoblasts) cultured cells and measuring the myotube diameter of treated cells (cells were the composition is added) and of control (untreated) cells, wherein the myoblasts are grown in non-inflammatory conditions, and wherein myotubes with a bigger diameter than untreated myotubes correspond to a composition comprising at least one androgen receptor agonist.

Another related non-claimed aspect of the present disclosure is an isolated non-steroid natural androgen-receptor agonist (being not urolithin B), possibly obtainable by the process of the present invention.

### Brief description of the figures

Figure 1 represents the comparative effect of urolithin A and urolithin B on the diameter of C2CI2 myotubes.
Figure 2 represents the morphological results of urolithin B addition on C2CI2 myotubes.
Figure 3 shows that Urolithin B induces mTOR and S6K1 phosphorylation.
Figure 4 shows that Urolithin B does not induce Akt or PRAS40 phosphorylation, but strongly reduces AMPK phosphorylation.
Figure 5 shows that Rapamycin blocks urolithin B-effect on the diameter of C2CI2 myotubes.
Figure 6 shows that Bicalutamide blocks urolithin B-effect on the diameter of C2CI2 myotubes.

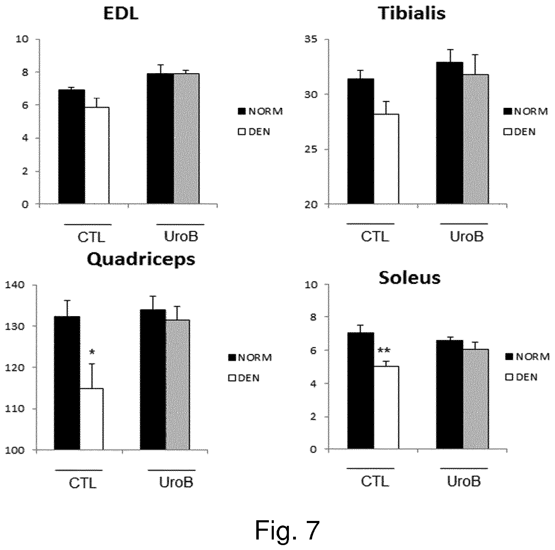

Figure 7 shows that intravenous urolithin B prevents in mammal the weight loss of denervated muscle.

### Detailed description of the invention

The inventors have searched for anti-inflammatory effect of several polyphenols on muscle cell anabolism, especially in the context of TNF-alpha-induced inflammation.
In this context, none of the tested compounds, except urolithin B, exhibited activity in significantly improving muscle cell growth and/or size.

However, urolithin B harbours weak antioxidant and/or anti-inflammatory properties.

Furthermore, the addition to muscle cells of urolithin B without the addition of TNF-alpha has resulted into improved muscle cell growth.

Therefore, these results can hardly be attributed to antioxidant and/or anti-inflammatory properties alleviating the TNF-alpha-induced stress.

The inventors then have elucidated the mode of action of urolithin B in enhancing muscle growth.

The inventors have found that urolithin B is an androgen-receptor agonist, but not the closely related urolithin A.

Therefore, urolithin B is a new molecule demonstrated as natural non-steroid androgen-receptor agonist.

This molecule can thus be used for the treatment or the prevention of osteoporosis (not part of the claimed invention), or as 'anabolisant' (possibly in synergy with other compounds for muscle growth and/or repair). Indeed, Urolithin B administration (to a patient) results into increased muscle size, a fact compatible with a label as anabolisant, while the mechanism of action comprises reduction of muscle catabolism.

Furthermore, polyphenols mixtures being extracts containing precursors of urolithin B, such as pomegranate extracts and/or ellagitannins are disclosed as hardly allowing for a sufficient concentration of urolithin B in the blood, at least if not taken regularly and/or at a (very) high dosage: urolithin B is not the only ellagitannin-derived compound; urolithin B is rather a minor component; the blood concentration of urolithin is patient-dependent, since depending on the gut flora of the patient.

Therefore, a first aspect of the present invention is composition (for instance a food (and/or a beverage), feed or a pharmaceutical composition) comprising at least 0.1% of urolithin B, where the % is by weight urolithin B: dry weight composition; and a sufficient amount of a compound selected from the group consisting of testosterone, leucine and/or creatine (and possibly a pharmaceutically or food or feed acceptable carrier)

A related aspect of the present disclosure is a food and/or a beverage or feed composition comprising a sufficient amount of urolithin B.

Urolithin B is preferred, for instance allowing injection or application through a dermal patch (such as over an injured muscle). In addition, even formulated for oral administration, urolithin B allows to apply (much) lower dose by comparison to urolithin B precursor, and the resulting blood content of urolithin B is independent of the patient's gut flora.

In the (food, feed and/or pharmaceutical) compositions of the present invention, urolithin B is advantageously present in an amount comprised between 0.1% % ([weight urolithin B]: dry weight of the composition) and (almost) 100%, preferably between (about) 1% and (about) 50%, still more preferably between (about) 10 % and (about) 25%.

In the (food, feed and/or pharmaceutical) compositions of the present invention, urolithin B is advantageously present in an amount comprised between (about) 1 % ([weight urolithin B ]: dry weight of the composition) and (almost) 100%, preferably between (about) 2% and (about) 50%, still more preferably between (about) 4 % and (about) 40% and even between (about) 10% (or (about) 15%,(about) 20%, (about) 25%) and (about) 30%).

The (food, feed and/or pharmaceutical) compositions of the present disclosure can be a mixture of (food, feed and/or pharmaceutical) compositions comprising urolithin B (such as between 0.1% % (by weight urolithin B : dry weight of the composition) and (almost) 100%, preferably between 1% and 50%, still more preferably between 10 % and 25%) and of (food, feed and/or pharmaceutical) compositions comprising urolithin B (such as between 1 % (by weight urolithin B: dry weight of the composition) and (almost) 100%, preferably between 2% and 50%, still more preferably between 4 % and 40% and even between 10% (or 15%, 20%, 25% and 30%).

Advantageously, the (food, feed and/or pharmaceutical) compositions of the present invention (comprising urolithin B) further comprise between (about) 1% (weight leucine: dry weight composition) and (about) 99%, preferably between (about) 5% and (about) 75%, more preferably between (about) 15% and (about) 50% of leucine.

Preferred (food, feed and/or pharmaceutical) compositions of the present invention comprise between 0.1%, (more preferably 0.5%) and (about) 10% of urolithin B, (weight urolithin B : dry weight composition) and comprises between (about) 2% (weight leucine: dry weight composition) and (about) 99% of leucine, preferably between (about) 5% and (about) 90%, more preferably between (about) 25% and (about) 75%.

Other preferred (food, feed and/or pharmaceutical) compositions of the present invention comprise between (about) 10% and (about) 25% urolithin B, (by weight urolithin B : dry weight composition) and comprises between (about) 2% (weight leucine: dry weight composition) and (about) 75% of leucine, preferably between (about) 5% and (about) 65%, more preferably between (about) 25% and (about) 60%.

Another possible food or feed composition of the present disclosure comprises between (about) 1% and (about) 75% of urolithin B (weight urolithin B: dry weight composition), and comprises between (about) 1% (weight leucine: dry weight composition) and (about) 99% of leucine, preferably between (about) 15% and (about) 75%, more preferably between (about) 25% and (about) 60%.

Another possible food or feed composition of the present disclosure comprise between (about) 25% and (about) 50% of urolithin B (weight urolithin B: dry weight composition), and comprises between (about) 2% (weight leucine: dry weight composition) and (about) 75% of leucine, preferably between (about) 15% and (about) 70%, more preferably between (about) 25% and (about) 60%.

Possibly, the leucine of food or feed compositions of the present invention is present in ingredients selected from the group consisting of plant extracts (such as soy hydrolysate, peas), (isolated) proteins, (partly) hydrolyzed proteins, branched amino acids (mixtures), isolated leucine and mixture thereof.

Thus possibly, further to the leucine, other amino acids including also taurine are present, especially in the food or feed compositions of the present invention, for instance in the form of added proteins, such as milk-proteins (whey), egg proteins, and/or in the form of added protein hydrolysates (e.g. whey hydrolysate, soy hydrolysate), possibly as full hydrolysate, and/or as isolated branched amino acids (e.g. mixtures consisting essentially of leucine, valine and isoleucine).

Alternatively, the amino acids of the (pharmaceutical) compositions of the present invention consists essentially of leucine.

Preferably, in the (food feed and/or pharmaceutical) compositions of the present invention (comprising urolithin B, and amino acids), the weight ratio of urolithin B to leucine (possibly present as plant extract, as protein, as protein hydrolysates or among the branched amino acids) is comprised between (about) 1:1 and (about) 1:30, preferably between (about) 1:2 and (about) 1:15, more preferably between (about) 1:4 and (about) 1:8.

Alternatively, in the (food feed and/or pharmaceutical) compositions of the present disclosure (comprising urolithin B and amino acids), the weight ratio of urolithin B to leucine (possibly present as plant extract, as protein, as protein hydrolysates or among the branched amino acids) is comprised between (about) 1:1 and (about) 1:30, preferably between (about) 1:2 and (about) 1:15, more preferably between (about) 1:4 and (about) 1:8.

Advantageously, the food or feed compositions of the present disclosure comprising urolithin B (and/or proteins such as whey or egg proteins and/or amino acids such as leucine) further comprise one or more additives selected from the group consisting of creatine, taurine, vitamins (preferably vitamin D), minerals (preferably Mg), isolated enzymes (preferably lactoferrin), polyphenols (ursolic acid, curcumin, resveratrol) and saccharides (such as glucose, fructose, sucrose and mixture thereof). For instance the compositions of the present disclosure comprise all of urolithin B and/or proteins and/or amino acids such as leucine), creatine and/or taurine, vitamins (preferably vitamin D), minerals (preferably Mg), and saccharides (such as glucose, fructose, sucrose), and possibly further comprises one or more of lactoferrin, ursolic acid, curcumin and resveratrol.

Advantageously, the food or feed compositions of the present disclosure comprising urolithin B (and/or proteins such as leucine) further comprise between (about) 30 mg and (about) 10 g of creatine, preferably between (about) 100 mg and (about) 5 g, more preferably between (about) 300 mg and (about) 4 g more preferably between (about) 1 g and (about) 3 g.

Preferably, in the (food feed and/or pharmaceutical) compositions of the present invention (comprising urolithin B), the weight ratio of urolithin B to creatine is comprised between (about) 1:1 and (about) 1:30, preferably between (about) 1:2 and (about) 1:15, more preferably between (about) 1:4 and (about) 1:8,

Advantageously, these food, feed and/or pharmaceutical compositions comprising a sufficient amount of urolithin B , and comprising leucine and one or more additive, is taken on a daily basis (or several times (e.g. 2-4) per day or per week (e.g. 2-14) for at least 6 months (to allow for accumulation upon the time of urolithin B in the blood; for instance reaching a blood content (in a human being or in an animal selected from the group consisting of pigs, sheep, cattle, dogs and horse of at least 10 nM, or even of at least 500 nM, 1 µM, 3 µM, 5 µM, 10 µM and up to 15 µM of urolithin B .

Advantageously, these food, feed and/or pharmaceutical compositions comprising a sufficient amount of urolithin B , and comprising leucine and one or more additive, and taken (by a human or by an animal selected from the group consisting of pigs, sheep, cattle, dogs and horse) on a daily basis for at least 6 months allows to reach blood level of urolithin B (independently of urolithin B precursor blood level, such as the blood level of urolithin A; this urolithin B precursor blood level being possibly (much) lower) comprised between (about) 10 nM and (about) 100 µM, preferably comprised between (about) 1 µM and (about) 50 µM, more preferably comprised between (about) 2.5 µM and (about) 15 µM.

Preferably, the sufficient amount of the urolithin B present in the pharmaceutical composition of the present invention is comprised between (about) 0.5 mg and (about) 1 g, preferably between (about) 5 mg and (about) 200 mg, more preferably between (about) 50 mg and (about) 100 mg.

Preferably, the sufficient amount of the urolithin B present in the pharmaceutical composition of the present disclosure is comprised between (about) 50 mg and (about) 5 g, preferably between (about) 100 mg and (about) 2 g, more preferably between (about) 200 mg and (about) 1 g.

Preferably, the sufficient amount of urolithin B present in the food or feed composition of the present invention is comprised between (about) 5 mg and (about) 2 Kgs, preferably between (about) 15 mg and (about) 50 g, more preferably between (about) 50 mg and (about) 10 g.

Preferably, the sufficient amount of urolithin B present in the food or feed composition of the present disclosure is comprised between (about) 5 mg and (about) 10 Kgs, preferably between (about) 200 mg and (about) 200 g, more preferably between (about) 500 mg and (about) 50 g.

Advantageously, this pharmaceutical composition comprising a sufficient amount of urolithin B is formulated for oral administration.

Alternatively, this pharmaceutical composition comprising a sufficient amount of urolithin B is formulated for non-oral administration (e.g. by injection or in a dermal patch).

Another preferred non-claimed aspect is a pharmaceutical composition comprising (i) a sufficient amount of urolithin B (i.e. at least 0.1 % or at least 0.5 % or even at least 1% ([w urolithin B : dry weight composition; or at least 5 mg (or at least 10 mg or even at least 50 mg) of urolithin B ), and (ii) from 0.01% (w: dry weight pharmaceutical composition) to (about) 20 % (w:w) of a compound selected from the group consisting of capsaicin, menthol, trolamine salicylate, camphor, methylsalicylate, for instance this pharmaceutical composition is for topical administration (on an injured muscle), such as in the form of a dermal patch (where urolithin B synergizes with the compound (ii) in healing the injured muscle).

A related aspect of the present invention is urolithin B or the above described food, feed or pharmaceutical compositions according to the invention for use in enhancing muscle growth (in the elderly) or in preventing or treating muscle myopathy (in a mammal subject, preferably a human patient, for instance in a human having be diagnosed as having a genetic disposition for myopathy).

A closely related non-claimed aspect of the present disclosure is urolithin B or the above described food, feed or pharmaceutical compositions according to the disclosure for use in the treatment or the prevention of osteoporosis in a mammal subject (preferably a human patient, such as a human patient diagnosed as suffering of osteoporosis or a human patient having a predisposition to osteoporosis).

The pharmaceutical composition comprising urolithin B for use in enhancing muscle growth (in the elderly) or in preventing or treating muscle myopathy in a (human) patient further comprises leucine (possibly in the form of plant extract, of proteins, of protein hydrolysates, of mixture of amino acids or of isolated leucine).

Preferably, the urolithin B content of these compositions (for use in enhancing muscle growth (in the elderly) or in preventing or treating muscle myopathy in a (human) patient) is comprised between 0.1% (by weight urolithin B:dry weight composition) and close to 100%, preferably between 0.5% and 75%, more preferably between 1% and 50%. The urolithin B content of these composition of the present disclosure (for use in enhancing muscle growth (in the elderly) or in preventing or treating muscle myopathy in a human patient) is comprised between 15% (by weight urolithin B:dry weight composition) and close to 100%, preferably between 20% and 75%, more preferably between 25% and 50%.

Still another related aspect of the present invention is urolithin B and leucine (possibly in the form of plant extract, of proteins, of protein hydrolysates, of mixture of amino acids or of isolated leucine) or the above described food, feed or pharmaceutical compositions according to the invention for use in enhancing muscle growth and/or size (in the ageing) or in preventing or treating muscle myopathy in a mammal subject, preferably a human patient.

Preferably, leucine (added in the pharmaceutical composition of the present invention and/or in the pharmaceutical composition for use in restoring muscle function or in preventing or treating muscle myopathy) is isolated, and/or is exogenous to an extract comprising urolithin B.

Advantageously, the leucine of the pharmaceutical composition (and/or in the pharmaceutical composition for use in restoring muscle function or in preventing or treating muscle myopathy) is present in an amount between (about) 1 mg and (about) 10 g, such as about 6 g of leucine.

Preferably, this pharmaceutical composition (and/or the pharmaceutical composition for use in restoring muscle function or in preventing or treating muscle myopathy further comprises creatine in an amount comprised between (about) 100 mg and (about) 10 g, more preferably between (about) 300 mg and (about) 5 g, still more preferably between (about) 1 g and (about) 3 g, and/or further comprises vitamins (preferably vitamin D), minerals (preferably Mg), and/or saccharides (such as glucose, fructose, sucrose), and possibly further comprises one or more of lactoferrin, ursolic acid, curcumin and resveratrol.

Still another related aspect of the present disclosure is a food or feed composition (an animal feed composition; preferably a feed composition for fish or for poultry or for mammals (horse, sheep, pigs, cattle, dogs)) comprising between (about) 5 mg and (about) 5 g of urolithin B, preferably between (about) 50 mg and (about) 1 g, more preferably between (about) 200 mg and (about) 500 mg, and possibly between (about) 1 g and (about) 15 g of leucine (preferably between (about) 2 g and (about) 10 g of leucine).

Preferably, the urolithin B content of this food or feed composition is comprised between (about) 0.1% (weight urolithin B: dry weight composition) and close to 100%, preferably between (about) 0.5% and (about) 75%, more preferably between (about) 1% and (about) 50%, still more preferably between (about) 10% and (about) 25%.

Still another related aspect of the present disclosure is a food or feed composition (an animal feed composition; preferably a feed composition for fish or for poultry or for mammals (horse, sheep, pigs, cattle, dogs, camel)) comprising between (about) 50 mg and (about) 5 g of urolithin B, preferably between (about) 200 mg and (about) 2 g, more preferably between (about) 500 mg and (about) 1 g, and possibly between (about) 1 g and (about) 15 g of leucine (preferably between (about) 2 g and (about) 10 g of leucine).

Preferably, the urolithin B content of this (animal) feed composition is comprised between (about) 1% (weight urolithin B: dry weight composition) and close to 100%, preferably between (about) 20% and (about) 75%, more preferably between (about) 25% and (about) 50%.

Preferably, animals are selected from the group consisting of pigs, sheep, cattle, dogs, horse and (less preferably) camel, poultry, or fish; hence the animal feed composition of the present invention is designed (quantitatively and qualitatively) for the selected animal, and is supplemented with urolithin B.

Still another related non-claimed aspect of the present disclosure is an isolated non-steroid natural androgen-receptor agonist.

Preferably, this isolated non-steroid natural androgen-receptor agonist is urolithin B.

Therefore another related aspect of the present disclosure is the (non-therapeutical) use of urolithin B for increasing muscle mass of a (non-diseased) human being or of a (non-diseased) animal selected from the group consisting of pigs, sheep, cattle, dogs, horse and (less preferably) camel, poultry or fish.

Preferably, the urolithin B content (in a composition) for increasing muscle mass is comprised between 0.1% (weight urolithin B:dry weight composition) and close to 100%, preferably between 0.5% and 75%, more preferably between 1% and 50%. Preferably, the urolithin B content (in a composition) of the present disclosure for increasing muscle mass is comprised between 15% (weight urolithin B:dry weight composition) and close to 100%, preferably between 20% and 75%, more preferably between 25% and 50%.

### Examples

### Comparative Example:

The inventors selected C2C12 murine skeletal muscle myoblasts for measuring the effect of polyphenols on muscle metabolism, especially when submitted to inflammatory stress (e.g. upon TNF-alpha exposure). In these conditions, anabolism is blocked.

Although rodents are not necessary suitable mammals (different pharmacokinetics of polyphenols derivatives than in human), isolated rodent cells can be used in the context of purified urolithin addition.

The inventors firstly selected urolithin A, as this metabolite was shown to have several advantageous properties; for instance, urolithin A is found in larger amount in blood of human beings than the other urolithins, and its anti-oxidant properties are bigger than those of urolithin B.

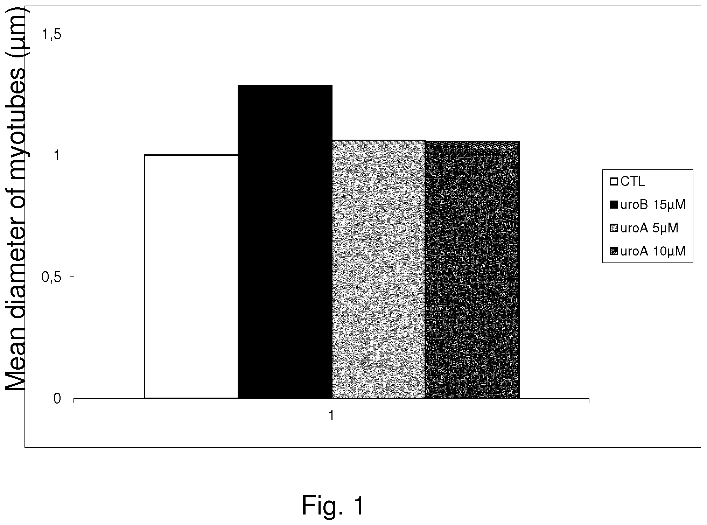

The addition of up to 10 µM of urolithin A does not increase the size of myotubes (see Figure 1, conditions 3 and 4 in function of the control condition 1).

### Example 1:

In order to further compare the anti-oxidant effect of urolithin towards muscle function, the inventors then selected urolithin B, which is the urolithin having the lowest antioxidant and anti-inflammatory properties.

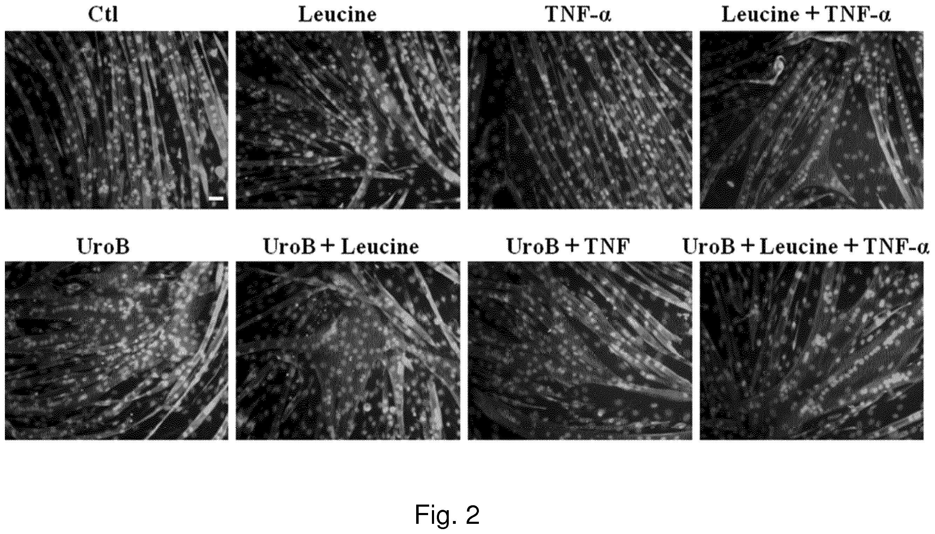

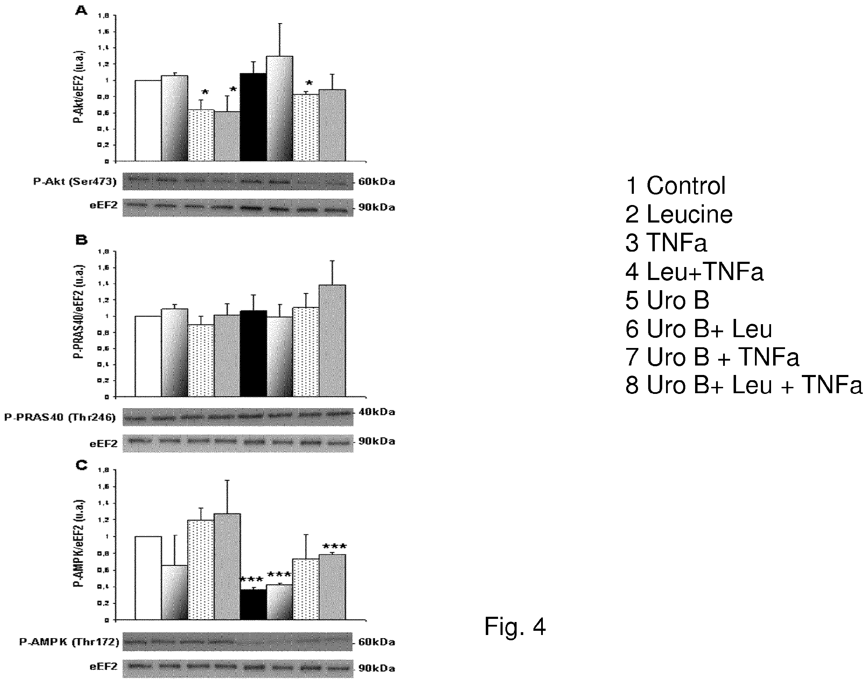

C2C12 myoblasts were differentiated for 4 days using 1% horse serum and 1% penicillin/streptomycin. The cells were thereafter grown for 24 h either with the same differentiation medium or with the differentiation medium supplemented with 15 µM urolithin B (conditions 5-8 of Figures 2-4 and of Table 1). Then the cells were grown either with the same differentiation medium or in the differentiation medium supplemented with 10 ng/mL TNF-alpha for 4 h (conditions 3-4 and 7-8 of Figures 2-4 and of Table 1). Thereafter, the cells were grown either with the same differentiation medium or in the differentiation medium supplemented with 5 mM leucine (conditions 2, 4, 6 and 8 of Figures 2-4 and of Table 1). The myotubes were labelled using anti-desmin antibodies, and nuclei labelling by Hoechst staining. Urolithin B consistently stimulates the diameter of myotubes.

The inventors have firstly measured if the addition of urolithin B presents some toxicity, but no toxicity was measured for concentrations of up to 40 µM.

The inventors have observed that urolithin B at 15 µM significantly increased the size of myotubes, and that this effect was resistant to the inflammatory stress (see Table 1, Figure 1 and Figures 2A&B). Furthermore, under inflammatory stress, urolithin B and leucine are synergic, even at these high doses.

**Table 1: mean diameter size of myotubes**

| | | |
|---|---|---|
| **Ctl** | | 19,04 |
| **Leucine** | | 25,5 |
| **TNFa** | | 16,7 |
| **Leucine + TNFa** | | 22,66 |
| **uroB** | | 32,6 |
| **uroB + Leucine** | | 28 |
| **uroB + TNFa** | | 29 |
| **uroB + TNFa + Leucine** | | 30,6 |

### Example 2:

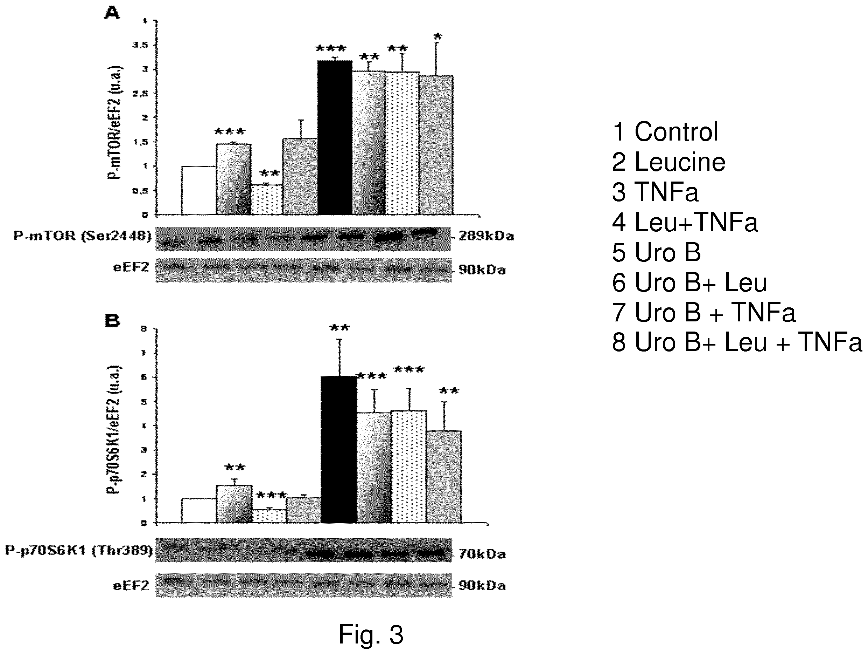

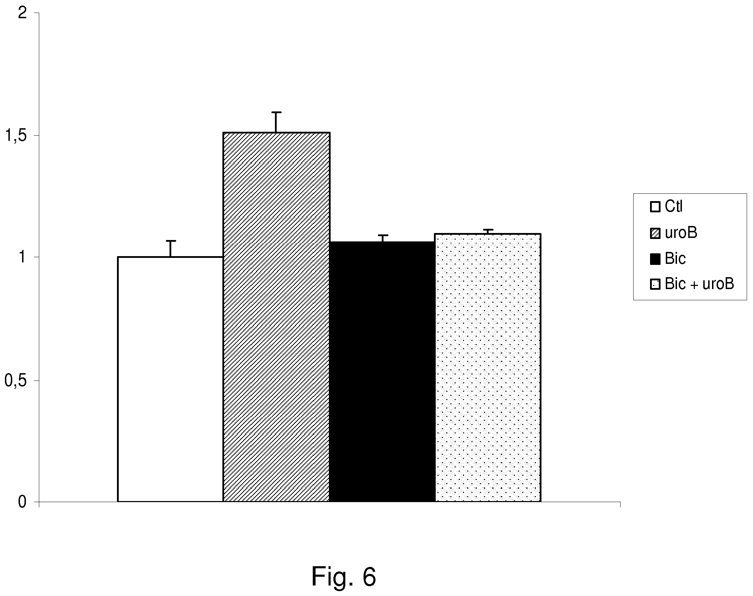

The inventors then refined the analysis in order to elucidate the underlining molecular pathway (Figures 3-6).

Among the pathways studied, the inventors have measured P70S6k phosphorylation to reflect protein synthesis and found a 5- to 6- fold induction upon urolithin B addition, but almost no induction upon urolithin A addition.

Surprisingly, co-addition of both urolithin A and B resulted into no induction of P70S6k phosphorylation. Therefore, such phenomenon is either due to toxicity of this specific composition or to an inhibitory effect of the urolithin B-induction by the presence of large amounts of urolithin A.

In other words, the inventors conclude that mixtures comprising urolithin B (and possibly other polyphenol derivatives) can only hardly have a positive effect on muscle anabolism. However, mixtures comprising urolithin B (and other polyphenol derivatives) can be advantageously combined with amino acids extracts (e.g. leucine) in order to boost the urolithin B-driven effect. Indeed, although this effect is low, since the effective urolithin B dose is reduced in these conditions and/or the specific urolithin B-effect is antagonized by its precursors, the low effect can be synergically boosted by the addition of specific amino acids, such as leucine.

### Example 3:

The inventors then have measured mTOR phosphorylation again to reflect protein synthesis and have observed a marginal leucine-induced phosphorylation and a strong urolithin B- induced phosphorylation.

The inventors then have measured Akt and PRAS40 phosphorylation still to analyse possible metabolism pathways. However, besides a tendency of synergy between the leucine and urolithin B addition, no major difference in phosphorylation patterns were observed.

On the other hand, the inventors have measured a strong reduction of AMPK phosphorylation upon urolithin B addition, again with a marginal bonus driven by leucine addition.

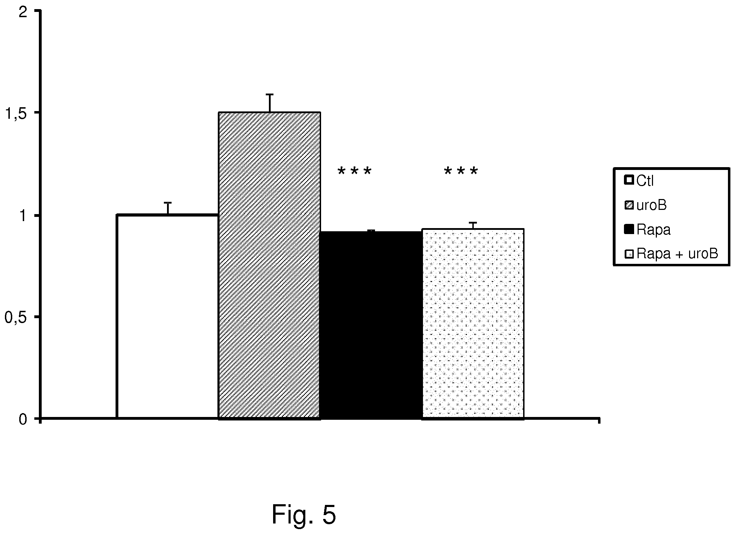

Finally, the inventors have measured the effect of rapamycin, on another mTOR inhibitor and of the anti-androgen bicalutamide (Figures 5-6 show the effect on the size of the muscular fibres) on the urolithin B-induced effects (including on the phosphorylation on mTOR, of AMPK,...) and conclude that urolithin B acts upon binding and activating the androgen receptor, then by releasing the AMPK-inhibition on mTOR, increases protein synthesis in muscle cells, then the diameter of muscle fibres.

## Claims

1. A composition comprising at least 0.1% of an androgen receptor agonist being urolithin B where the % is by weight urolithin B: dry weight composition; and
a sufficient amount of a compound selected from the group consisting of testosterone, leucine and/or creatine.

2. The composition of claim 1 comprising at least 0.5% of urolithin B.

3. The composition according to claim 1 or claim 2, comprising at least 5 % of leucine where the % is by weight leucine:dry weight of the composition.

4. The composition according to any of the preceding claims comprising at least 100 mg of creatine.

5. The composition according to any of the preceding claims, being an edible composition.

6. The composition of claim 5 wherein the amount of urolithin B is sufficient for obtaining urolithin B content of at least 100 nM in the blood of a mammal.

7. The composition according to any of the preceding claims further comprising one or more additives selected from the group consisting of vitamin D, magnesium, lactoferrin, ursolic acid, hydroxymethyl butyrate and mixtures thereof.

8. The composition according to any one of the preceding claims for therapeutic use in enhancing muscle size in an elder human patient.

9. The composition according to any one of the preceding claims for use in the prevention or treatment of myopathy.

10. The composition according to claim 9 for use in the prevention or treatment of the myopathy of human patient having been diagnosed as having a genetic disposition for myopathy.

11. An edible pharmaceutical composition comprising an adequate pharmaceutical carrier and an androgen receptor agonist being urolithin B wherein the amount of the said urolithin B is of at least 10% by weight urolithin B: dry weight pharmaceutical composition and further comprises at least 0,25g of leucine and/or at least 0,1g of creatine.

12. An injectable or topical pharmaceutical composition comprising an adequate pharmaceutical carrier and an androgen agonist being urolithin B wherein the amount of the said urolithin B is of at least 1 mg or of at least 0.1% by weight urolithin B: dry weight pharmaceutical composition and further comprises at least 0,25g of leucine and/or at least 0,1g of creatine.

13. The pharmaceutical composition of claim 12 which is incorporated into a patch.

14. The pharmaceutical composition of claim 12 or claim 13 further comprising at least 0.01 % by weight compound: dry weight pharmaceutical composition where the compound is selected from the group consisting of capsaicin, menthol, trolamine salicylate, camphor, methylsalicylate, and hydroxymethyl butyrate.

15. A kit of parts comprising an androgen receptor agonist being urolithin B together with other compounds for muscle growth and/or repair, and wherein said other compounds are testosterone and hydroxymethyl butyrate.

16. A composition comprising urolithin B, together with testosterone and hydroxymethyl butyrate for therapeutic use in enhancing muscle size in an elder human patient or in the prevention or treatment of myopathy.

17. Non-therapeutic use of a composition comprising an androgen receptor agonist being urolithin B, together with testosterone and hydroxymethyl butyrate for enhancing muscle size in a mammal.

## Patentansprüche

1. Zusammensetzung, umfassend mindestens 0,1 % eines Androgenrezeptor-Agonisten, der Urolithin B ist, wobei der % dem Gewicht nach Urolithin B: Trockengewichtszusammensetzung ist; und
eine ausreichende Menge einer Verbindung, ausgewählt aus der Gruppe bestehend aus Testosteron, Leucin und/oder Kreatin.

2. Zusammensetzung nach Anspruch 1, umfassend mindestens 0,5 % Urolithin B.

3. Zusammensetzung nach Anspruch 1 oder Anspruch 2, umfassend mindestens 5 % Leucin, wobei der % dem Gewicht nach das Leucin:Trockengewicht der Zusammensetzung ist.

4. Zusammensetzung nach einem der vorhergehenden Ansprüche, umfassend mindestens 100 mg Kreatin.

5. Zusammensetzung nach einem der vorhergehenden Ansprüche, die eine essbare Zusammensetzung ist.

6. Zusammensetzung nach Anspruch 5, wobei die Menge an Urolithin B ausreicht, um einen Urolithin-B-Gehalt von mindestens 100 nM im Blut eines Säugers zu erhalten.

7. Zusammensetzung nach einem der vorhergehenden Ansprüche, ferner umfassend einen oder mehrere Zusatzstoffe, ausgewählt aus der Gruppe bestehend aus Vitamin D, Magnesium, Lactoferrin, Ursolsäure, Hydroxymethylbutyrat und Mischungen davon.

8. Zusammensetzung nach einem der vorhergehenden Ansprüche zur therapeutischen Verwendung beim Steigern von Muskelgröße bei einem älteren menschlichen Patienten.

9. Zusammensetzung nach einem der vorhergehenden Ansprüche zur Verwendung bei der Vorbeugung oder Behandlung von Myopathie.

10. Zusammensetzung nach Anspruch 9 zur Verwendung bei der Vorbeugung oder Behandlung der Myopathie eines menschlichen Patienten, bei dem eine genetische Veranlagung für Myopathie diagnostiziert wurde.

11. Essbare pharmazeutische Zusammensetzung, umfassend einen geeigneten pharmazeutischen Träger und einen Androgenrezeptor-Agonisten, der Urolithin B ist, wobei die Menge des Urolithin B mindestens 10% dem Gewicht nach Urolithin B: Trockengewicht der pharmazeutischen Zusammensetzung ist und ferner mindestens 0,25 g Leucin und/oder mindestens 0,1 g Kreatin umfasst.

12. Injizierbare oder topische pharmazeutische Zusammensetzung, umfassend einen geeigneten pharmazeutischen Träger und einen Androgenagonisten, der Urolithin B ist, wobei die Menge des Urolithin B mindestens 1 mg oder mindestens 0,1 % dem Gewicht nach Urolithin B: Trockengewicht der pharmazeutischen Zusammensetzung ist und ferner mindestens 0,25 g Leucin und/oder mindestens 0,1 g Kreatin umfasst.

13. Pharmazeutische Zusammensetzung nach Anspruch 12, die in ein Pflaster eingearbeitet ist.

14. Pharmazeutische Zusammensetzung nach Anspruch 12 oder Anspruch 13, ferner umfassend mindestens 0,01% dem Gewicht nach Verbindung: Trockengewicht der pharmazeutischen Zusammensetzung, wobei die Verbindung ausgewählt ist aus der Gruppe bestehend aus Capsaicin, Menthol, Trolaminsalicylat, Campher, Methylsalicylat und Hydroxymethylbutyrat.

15. Teilesatz, umfassend einen Androgenrezeptor-Agonisten, der Urolithin B ist, zusammen mit anderen Verbindungen für Muskelwachstum und/oder -reparatur, und wobei die anderen Verbindungen Testosteron und Hydroxymethylbutyrat sind.

16. Zusammensetzung, umfassend Urolithin B zusammen mit Testosteron und Hydroxymethylbutyrat zur therapeutischen Verwendung beim Steigern von Muskelgröße bei einem älteren menschlichen Patienten oder bei der Vorbeugung oder Behandlung von Myopathie.

17. Nichttherapeutische Verwendung einer Zusammensetzung, umfassend einen Androgenrezeptor-Agonisten, der Urolithin B ist, zusammen mit Testosteron und Hydroxymethylbutyrat zum Steigern von Muskelgröße bei einem Säuger.

## Revendications

1. Composition comprenant au moins 0,1 % d'un agoniste du récepteur androgène étant l'urolithine B où le % est en poids d'urolithine B : composition en poids sec ; et
une quantité suffisante d'un composé choisi dans le groupe constitué par testostérone, leucine et/ou créatine.

2. Composition selon la revendication 1 comprenant au moins 0,5 % d'urolithine B.

3. Composition selon la revendication 1 ou la revendication 2, comprenant au moins 5 % de leucine où le % est en poids de leucine : composition en poids sec.

4. Composition selon l'une quelconque des revendications précédentes comprenant au moins 100 mg de créatine.

5. Composition selon l'une quelconque des revendications précédentes, étant une composition comestible.

6. Composition selon la revendication 5 dans laquelle la quantité d'urolithine B est suffisante pour obtenir une teneur en urolithine B d'au moins 100 nM dans le sang d'un mammifère.

7. Composition selon l'une quelconque des revendications précédentes comprenant en outre un ou plusieurs additifs choisis dans le groupe constitué par vitamine D, magnésium, lactoferrine, acide ursolique, butyrate d'hydroxyméthyle et des mélanges de ceux-ci.

8. Composition selon l'une quelconque des revendications précédentes pour une utilisation thérapeutique dans l'amélioration de la masse musculaire chez un patient humain âgé.

9. Composition selon l'une quelconque des revendications précédentes pour une utilisation dans la prévention ou le traitement de la myopathie.

10. Composition selon la revendication 9 pour une utilisation dans la prévention ou le traitement de la myopathie chez un patient humain ayant été diagnostiqué comme ayant une prédisposition génétique à la myopathie.

11. Composition pharmaceutique comestible comprenant un support pharmaceutique adéquat et un agoniste du récepteur androgène étant l'urolithine B, dans laquelle la quantité de ladite urolithine B est d'au moins 10 % en poids d'urolithine B : composition pharmaceutique en poids sec et comprend en outre au moins 0,25 g de leucine et/ou au moins 0,1 g de créatine.

12. Composition pharmaceutique injectable ou topique comprenant un support pharmaceutique adéquat et un agoniste des androgènes étant l'urolithine B, dans laquelle la quantité de ladite urolithine B est d'au moins 1 mg ou d'au moins 0,1 % en poids d'urolithine B : composition pharmaceutique en poids sec et comprend en outre au moins 0,25 g de leucine et/ou au moins 0,1 g de créatine.

13. Composition pharmaceutique selon la revendication 12 qui est incorporée dans un patch.

14. Composition pharmaceutique selon la revendication 12 ou la revendication 13 comprenant en outre au moins 0,01 % en poids de composé : composition pharmaceutique en poids sec, où le composé est choisi dans le groupe constitué par capsaicine, menthol, salicylate de trolamine, camphre, méthylsalicylate et butyrate d'hydroxyméthyle.

15. Kit de pièces comprenant un agoniste du récepteur androgène étant l'urolithine B conjointement avec d'autres composés pour la croissance et/ou la réparation des muscles et dans lequel lesdits autres composés sont la testostérone et le butyrate d'hydroxyméthyle.

16. Composition comprenant de l'urolithine B, conjointement avec de la testostérone et du butyrate d'hydroxyméthyle pour une utilisation thérapeutique dans l'amélioration de la masse musculaire chez un patient humain âgé ou dans la prévention ou le traitement de la myopathie.

17. Utilisation non thérapeutique d'une composition comprenant un agoniste du récepteur androgène étant l'urolithine B, conjointement avec de la testostérone et du buryrate d'hydroxyméthyle pour améliorer la masse musculaire chez un mammifère.
